# FASCICULE DE BREVET EUROPEEN

(11) **EP 1 623 005 B1**
(45) Date de publication et mention de la délivrance du brevet: **01.08.2007**
(21) Numéro de dépôt: 04742691.1
(22) Date de dépôt: 10.05.2004
(51) Int. Cl.: C12M 1/04, C12M 1/18, C12M 1/00, C12N 1/00

(54) **PROCEDE DE PREPARATION D'UNE SUSPENSION DE MICRO-ORGANISMES**
VERFAHREN ZUR VORBEREITUNG EINER SUSPENSION VON MIKROORGANISMEN
METHOD FOR PREPARATION OF A SUSPENSION OF MICRO-ORGANISMS

(30) Priorité: 13.05.2003 FR 0305732
(43) Date de publication de la demande: 08.02.2006
(73) Titulaire: DANISCO A/S, 1411 Copenhagen K. (DK)
(72) Inventeur: GUILLAUD, Denis, F-38850 Paladru (FR); FONTAINE, Eloi, F-37000 Tours (FR)
(74) Mandataire: Touati, Catherine
(86) Numéro de dépôt international: PCT/FR2004/001135
(87) Numéro de publication internationale: WO 2004/101732

(56) Documents cités:
- US-A1- 2002 110 915

## Description

La présente invention a pour objet un procédé pour préparer une suspension de micro-organismes dans un liquide utilisant un réservoir scellé comprenant 2 chambres dans lesquelles on introduit alternativement un gaz de manière à créer un flux dudit liquide. L'invention a également pour autre objet un procédé d'ensemencement dudit micro-organisme.

US-A-2002110915 décrit un bioréacteur et ses variantes ainsi que des façons de l'utiliser.

Les micro-organismes sont utilisés dans de nombreuses industries, notamment dans l'industrie agro-alimentaire. Ils sont utilisés entres autres pour fermenter, aromatiser, affiner ou texturer les aliments, notamment les produits laitiers ou les produits de charcuterie. Ils sont également utilisés pour protéger le milieu dans lesquels ils sont incorporés contre les contaminations par d'autres micro-organismes et également utilisés pour leur effet probiotiques.

Selon les applications, les micro-organismes sont commercialisés sous forme sèche, lyophilisée, congelée ou sous forme de suspension et sont utilisés le plus souvent sous forme de suspension. Dans le cas des micro-organismes sous forme sèche, lyophilisée ou congelée, leur utilisation nécessite une mise en suspension préalable. Cette mise en suspension est généralement réalisée dans des cuves, mélangeurs ou réacteurs dont les contenances varient de 1 à 5000 litres.

Les cuves ou mélangeurs actuellement disponibles comprennent généralement une cuve en verre ou en inox ainsi qu'un système d'agitation sous la forme d'une hélice montée au fond de la cuve pour agiter le milieu liquide.

Après chaque opération de mélange, ces cuves ou mélangeurs doivent être lavés, décontaminés et rincés. Ils sont éventuellement stérilisés avant chaque nouveau mélange.

Les opérations de lavage, de maintenance et de stérilisation de ces réacteurs sont des étapes longues, qui impliquent de nombreuses manipulations et des coûts supplémentaires. En termes de coût, de temps et de moyens humains, elles peuvent représenter jusqu'à 30% de l'exploitation du réacteur, ce qui est très élevé.

De plus le lavage de ces réacteurs peut entraîner des problèmes de contamination si ce lavage n'est pas suffisamment efficace.

De plus dans le cas particulier de la mise en suspension de micro-organismes, les micro-organismes ne doivent pas coller aux parois de la cuve ou s'agglomérer au fond de la cuve par décantation. De plus il est essentiel d'éviter toute contamination de la suspension par le milieu environnant.

Afin de répondre aux exigences des industriels, il est devenu nécessaire de trouver un procédé pour préparer une suspension de micro-organismes qui permette de prévenir les contaminations bactériennes, tout en assurant un mélange homogène, à taille industrielle. Par le terme taille industrielle on entend un procédé qui permette de mélanger de gros volumes et qui puisse être utilisé sur une ligne de production.

Aussi le problème que se propose de résoudre la présente invention est de fournir un procédé pour préparer une suspension homogène de micro-organismes qui évite les contaminations, et soit à taille industrielle.

Dans ce but l'invention propose un procédé pour préparer une suspension de micro-organismes dans un liquide caractérisé en ce que l'on utilise un réservoir scellé (1) comprenant :
- 2 chambres communiquant par la partie inférieure du réservoir,
- au moins un orifice (2) d'introduction d'un liquide dans une chambre,
- chaque chambre comprenant au moins un orifice (3) d'admission et au moins un orifice (4) d'évacuation d'un gaz
- le réservoir contenant en outre les micro-organismes sous forme solide (8)
et caractérisé en ce que l'on introduit le liquide par l'orifice d'introduction dans au moins une des chambres, puis l'on introduit alternativement un gaz par lesdits orifices d'admission dans une chambre puis dans l'autre chambre de manière à créer un flux dudit liquide en va et vient dans chaque chambre.

L'invention propose également un procédé d'ensemencement caractérisé en ce qu'on utilise une suspension préparée par le procédé cité précédemment.

Le procédé selon l'invention a pour avantage de pouvoir être mis en oeuvre dans toutes industries utilisant des micro-organismes, notamment l'industrie agro-alimentaire, pharmaceutique, cosmétique, alimentaire, l'agriculture, ainsi que dans les domaines de la nutrition animale, des aliments pour animaux et de l'hygiène au sens large en particulier l'hygiène corporelle (par exemple les dentifrices) ou l'hygiène industrielle.

L'utilisation du réservoir selon l'invention permet de fournir une suspension de micro-organismes qui soit homogène et dans des conditions qui évitent toutes contaminations par le milieu extérieur.

Le procédé selon l'invention a encore pour avantage de réduire les phénomènes de décantation et d'éviter que les micro-organismes ne se concentrent dans le fond du réservoir.

L'invention sera mieux comprise et d'autres caractéristiques, détails et avantages de celle-ci apparaîtront plus clairement au cours de la description explicative qui va suivre, faite en référence aux dessins schématiques annexés donnés uniquement à titre d'exemples et dans lesquels :
- la figure 1 est une vue de face du réservoir utilisé selon l'invention;
- la figure 2 est une vue de face du même réservoir en cours de fonctionnement et contenant la suspension;
- la figure 3 est une vue de dessus de ce même réservoir.

La présente invention a tout d'abord pour objet un procédé pour préparer une suspension de micro-organismes dans un liquide caractérisé en ce que l'on utilise un réservoir scellé (1) qui va maintenant être décrit plus en détail.

Ce réservoir (1) comprend 2 chambres (1a) et (1b) communiquant par la partie inférieure du réservoir (1 c).

Au moins une des chambres comprend en outre au moins un orifice (2), de préférence deux, d'introduction d'un liquide dans une chambre.

Chaque chambre comprend au moins un orifice (3) d'admission et au moins un orifice (4) d'évacuation d'un gaz.

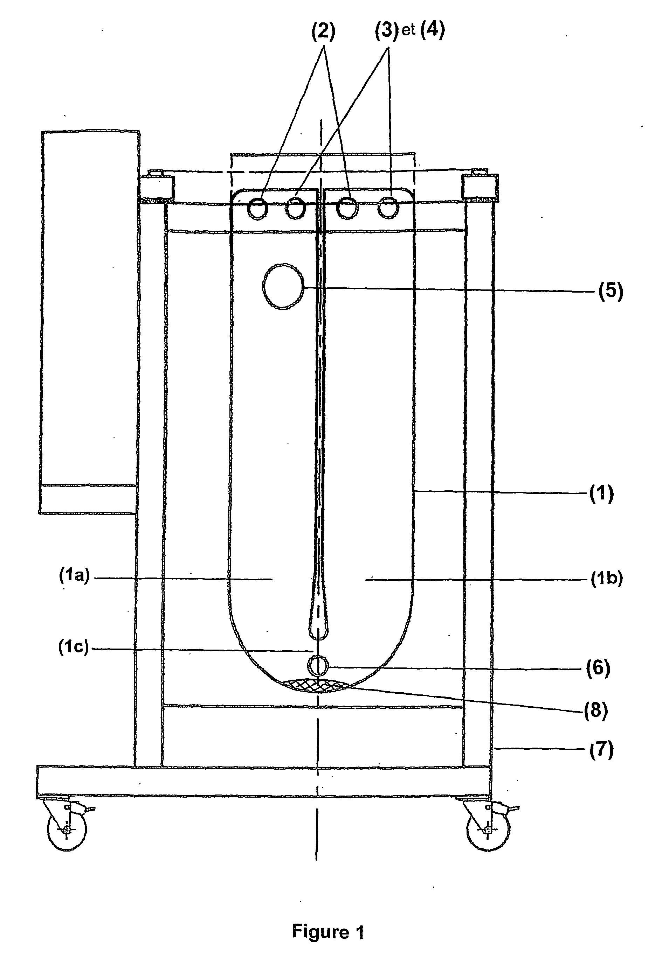

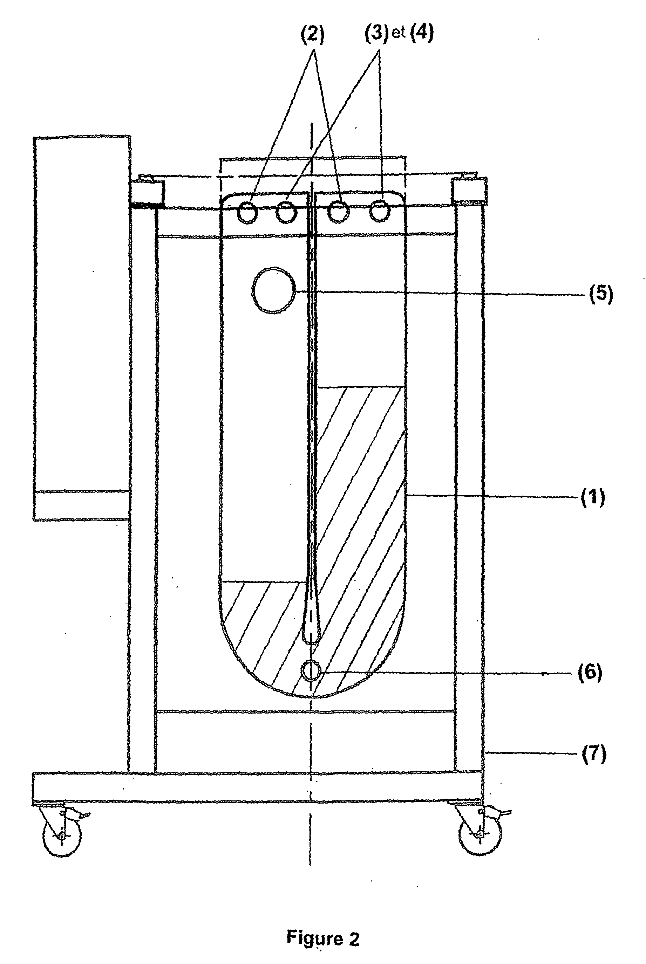

Ces orifices sont de préférence localisés dans la partie supérieure du réservoir, comme on le voit sur les figures 1 et 2.

Ces orifices peuvent être munis d'un clapet, d'un opercule, d'un robinet, d'une valve, d'une vanne ou de tout moyen destiné à réguler le mouvement d'un fluide dans ces orifices.

De préférence, le réservoir selon l'invention comprend des orifices (3) et (4) qui permettent à la fois l'admission et l'évacuation de gaz alternativement, comme le montre la figure 1.

Le réservoir contient en outre les microorganismes sous forme solide (8).

Les chambres (1a) et (1 b) du réservoir sont similaires ou identiques. Elles ont les mêmes dimensions et la même capacité volumique.

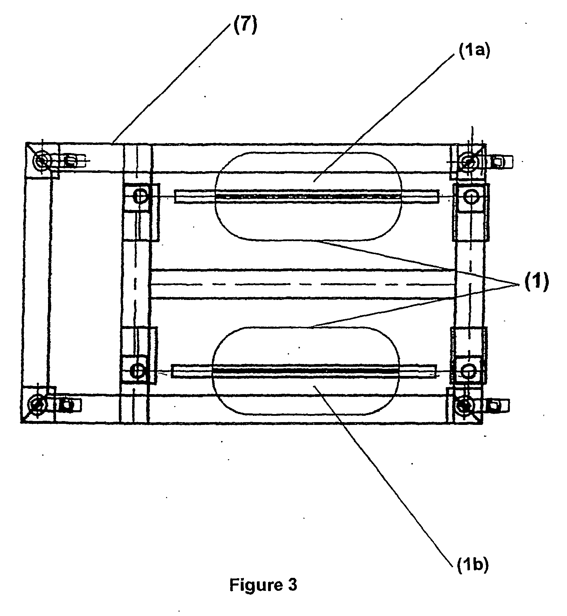

La forme de ces chambres (1a) et (1b) peut être quelconque et de section cylindrique, circulaire ou rectangulaire avec extrémités arrondies comme le montre la figure 3.

De préférence, le réservoir selon l'invention comprend 2 chambres disposées sensiblement en forme de U.

Dans ce cas particulier, chaque chambre (1a) et (1b) constitue un bras du U et la partie inférieure (1 c) du réservoir constitue la partie arrondie du U. Par disposées sensiblement en forme de U, on entend une disposition se rapprochant de la forme du U, et dans laquelle les axes des 2 chambres sont parallèles ou pratiquement parallèles. Les axes des deux chambres peuvent former un angle plus important et dans ce cas le réservoir présente une forme se rapprochant ainsi d'une forme en V.

Le réservoir est une poche scellée, avantageusement scellée dans la partie supérieure du réservoir, de préférence dans la partie haute des orifices.

Le réservoir selon l'invention peut être avantageusement une poche unique repliée sur elle-même de manière à former 2 chambres distinctes, (1 a) et (1 b) comme le montre les figures 1 et 2. Dans ce cas, le réservoir scellé comprendra 2 chambres disposées sensiblement en forme de U et dont les bras du U sont très proches, voire en contact, comme on peut le voir sur la figure 1.

Le réservoir préféré, pour mettre en oeuvre le procédé selon l'invention, peut être un réservoir scellé flexible ou souple, notamment en matière plastique. Il est également possible d'envisager un réservoir scellé rigide, notamment en inox ou en plastique rigide.

Ce réservoir est constitué de préférence d'une matière flexible ou souple comme par exemple le polypropylène, le polyester, le polyamide, la cellulose ou de tout autre matériel flexible compatible avec les produits alimentaires. De préférence le réservoir est en polyéthylène.

On utilisera un matériau souple dans le cas d'un réservoir qui est replié sur lui-même.

Le réservoir (1) selon l'invention peut comprendre plus particulièrement au moins un orifice (5) de remplissage permettant d'introduire les micro-organismes sous forme solide. En particulier il permet d'introduire une poudre dans le réservoir. La poudre introduite est de préférence une poudre contenant des micro-organismes ou une poudre de micro-organismes à suspendre dans un liquide.

Il est à noter que le micro-organisme sous forme solide (8) peut comprendre en outre des polysaccharides, des enzymes, des minéraux, des protéines, des sucres, des composés azotés, des facteurs de croissances et tous composés habituellement présents en mélange avec des micro-organismes.

Le réservoir (1) selon l'invention peut comprendre au moins un orifice (6) de vidange dudit réservoir permettant de vider le réservoir.

L'orifice (6) est de préférence localisé dans la partie inférieure du réservoir.

Les orifices (5) et (6) précédents peuvent être muni d'un clapet, d'un opercule, d'un robinet, d'une valve, d'une vanne ou de tout moyen destiné à réguler le mouvement d'un solide ou d'un fluide dans ces orifices.

La capacité du réservoir selon l'invention est variable et en particulier elle peut être comprise entre 5 litres et 100 litres, de préférence comprise entre 10 litres et 50 litres. De plus pour un bon fonctionnement du réservoir, la partie (1 c) doit présenter une section verticale d'au moins 15 cm.

A titre de micro-organismes, on entend par exemples les levures, les bactéries, notamment les bactéries lactiques, et plus généralement tous les micro-organismes utilisés dans l'industrie agro-alimentaire, pharmaceutique, cosmétique, alimentaire, l'agriculture, ainsi que dans les domaines de la nutrition animale, des aliments pour animaux et de l'hygiène au sens large en particulier l'hygiène corporelle (par exemple les dentifrices) ou l'hygiène industrielle. Ces micro-organismes sont utilisés seuls ou en mélanges.

Parmi les bactéries les plus utilisées et qui sont présents dans les ferments, on peut citer celles appartenant aux genres *Lactococcus, Streptococcus, Lactobacillus, Leuconostoc, Pediococcus, Bifidobacterium, Brevibacterium, Carnobacrerium, Enterococcus, Micrococcus, Vagococcus, Staphylococcus, Bacillus, Kocuria, Arthrobacter* et *Corynebacterium.*

Il est à noter que le réservoir est de manière avantageuse fixé par sa partie supérieure sur une potence (7), notamment une potence mobile. Cette potence peut-être déplacée, lorsqu'elle est montée par exemple comme sur la figure 1 sur roulettes, vers toutes les parties de la chaîne industrielle, avant, après ou pendant tout le procédé industriel.

Par potence mobile, on entend de préférence un châssis, métallique ou non, sur lequel est suspendu le réservoir selon l'invention. Le réservoir est de préférence suspendu verticalement.

Lorsque l'on suspend le réservoir, les micro-organismes sous forme solide (8) se retrouve de préférence dans la partie (1c) du réservoir.

Le réservoir pour mettre en oeuvre le procédé selon l'invention, peut aussi être un réservoir scellé jetable. Notamment dans le cas d'un réservoir scellé en matière plastique, celui-ci peut être à usage unique et jetable après utilisation.

Il est également possible de prévoir de stériliser le réservoir scellé avant l'introduction des micro-organismes (8). Cette stérilisation peut se faire par exemple par rayonnement UV ou gamma.

Le fonctionnement du réservoir va maintenant être décrit.

Tout d'abord le réservoir est rempli avec les micro-organismes sous forme solide (8). Il est noter à ce stade que l'on peut aussi ajouter d'autres composés additionnels, qui sont décrit plus haut (polysaccharides, des enzymes, des minéraux, des protéines, des sucres, des composés azotés, des facteurs de croissances ou tous composés habituellement présents en mélange avec des micro-organismes) et ceci sous n'importe quelle forme.

Au cours du remplissage, le réservoir peut être sous forme dépliée ou repliée, ou dans la position suspendue. Si le réservoir était sous forme déplié pour le remplissage, il est ensuite plié sur lui-même sensiblement en forme de U et suspendu à la potence.

Le procédé selon l'invention utilise de préférence un réservoir qui peut être mis en oeuvre pour la mise en suspension de micro-organismes dans un milieu liquide. Cette mise en suspension est réalisée grâce à l'agitation dudit liquide en présence du micro-organisme sous forme solide, en particulier sous forme de poudre, dans le réservoir.

Au moins un des orifices (2) d'introduction permet l'arrivée dudit liquide dans le réservoir scellé.

Avantageusement, le liquide introduit est un milieu aqueux et plus particulièrement de l'eau, de l'eau contenant des agents stabilisants, un milieu lacté ou à base de lait ou des jus de fruits.

Le milieu aqueux est préalablement stérilisé, de préférence il est filtré sur membrane d'au plus 0,45 µm, plus particulièrement au plus 0,22 µm. Il est à noter que l'on peut utiliser l'eau du robinet.

Avantageusement la température du milieu aqueux à son arrivée dans le réservoir scellé, est comprise entre 5°C et 50°C, de préférence comprise entre 8°C et 20°C.

Le procédé selon l'invention utilise un gaz qui est introduit dans au moins l'une des chambres par au moins un des orifices (3) qui permet l'arrivée de gaz dans le réservoir (1).

Le terme gaz au sens de la présente invention inclut également l'air.

On utilise avantageusement de l'air ou un gaz qui n'intervient pas dans la respiration et / ou l'oxydation des micro-organismes, des ferments et des bactéries.

Le gaz injecté peut être un gaz chimiquement et biologiquement inerte, de préférence on injecte de l'air, de l'argon, plus particulièrement de l'azote ou du dioxyde de carbone ou leurs mélanges.

Par gaz biologiquement inerte, on entend un gaz qui n'intervient pas dans la multiplication et la dégradation des micro-organismes.

Tout d'abord l'admission de gaz se fait dans une chambre, par exemple la chambre (1a). Cette admission provoque la baisse du niveau du liquide dans la chambre (1a). L'admission de gaz dans la chambre (1a) par l'orifice (3), est aussitôt suivi de l'évacuation, par l'orifice (4), du gaz se trouvant au-dessus du niveau dudit liquide présent dans la chambre (1b) ce qui provoque l'élévation du niveau du liquide dans la chambre (1 b). Puis on procède à l'arrivée de gaz par l'orifice (3) dans la chambre (1b), ce qui provoque une poussée verticale sur le liquide. Cette poussée provoque le transfert du liquide de la chambre (1b) vers la chambre (1a) et est aussitôt suivie de l'évacuation du gaz se trouvant au-dessus du niveau dudit liquide présent dans la chambre (1a).

L'agitation est réalisée par injection successive de gaz dans chaque chambre, ce qui crée une poussée verticale appliquée sur le liquide qui va permettre le transfert de la suspension d'une chambre à l'autre du réservoir.

La figure 2 montre un réservoir selon l'invention en cours de fonctionnement où le liquide est transféré de la première chambre vers la deuxième chambre sous l'effet de la poussée verticale.

L'admission de gaz dans l'une quelconque des chambres est de préférence déclenchée automatiquement grâce à 2 cellules optiques situées de par et d'autre des chambres. Ces cellules déclenchent l'admission de gaz lorsque le faisceau optique est par exemple rompu.

La pression de gaz dans le réservoir scellé, au cours de l'agitation, est inférieure à 5 bars, plus particulièrement inférieure à 1 bar.

La pression de gaz est de préférence déterminée en fonction des performances du matériau utilisé pour fabriquer le réservoir.

L'injection de gaz peut également se faire par intervalle régulier de temps entre chaque admission de gaz. De préférence l'intervalle de temps entre chaque admission de gaz est compris entre 0,5 minutes et 30 minutes.

L'agitation permet la mise en suspension des micro-organismes dans le milieu liquide.

Lorsque la suspension aqueuse de micro-organismes est réalisée, il est possible de vidanger le réservoir scellé de manière aseptique par l'orifice (6) de vidange.

Ce moyen de vidange est avantageusement placé au fond du réservoir, mais il peut également être placé à d'autres endroits du réservoir en fonction des diverses applications.

Cette vidange est réalisée par injection de gaz ou d'air à l'intérieur du réservoir scellé par au moins un orifice (3), ou par gravité de la suspension aqueuse de micro-organismes.

Selon une application avantageuse, le procédé selon l'invention utilise ledit réservoir pour ensemencer un milieu laitier avec une suspension de micro-organismes.

L'invention a aussi pour objet un procédé d'ensemencement caractérisé en ce qu'on utilise une suspension de micro-organismes préparée par le procédé décrit ci-dessus.

Plus particulièrement, il s'agit d'un procédé d'ensemencement d'un milieu laitier, mais d'autres milieux peuvent être envisagés, mentionnés ci-après.

Par exemple, la suspension de micro-organismes peut être un ferment qui peut être introduit dans le milieu à ensemencer, notamment un milieu lacté de manière à déclencher la fermentation dudit milieu lacté et / ou la texturation de ce milieu.

L'ensemencement d'un milieu lacté par une suspension de micro-organismes est réalisé avantageusement à un débit compris entre 10 ml/min et 1000 ml/min, de préférence compris entre 100 ml/min et 500 ml/min. Le débit est régulé par une pompe ou par l'injection de gaz dans le réservoir.

Cet ensemencement se fait à une température comprise avantageusement entre 5°C et 50°C, de préférence comprise entre 8°C et 45°C.

L'ensemencement du milieu lacté par la suspension de micro-organismes se fait sur une période pouvant s'étendre jusqu'à 72 heures, plus particulièrement sur une période pouvant s'étendre jusqu'à 48 heures, préférentiellement sur une période pouvant s'étendre jusqu'à 24 heures.

Il est possible d'ensemencer le milieu lacté par mise en oeuvre simultanée ou différée de plusieurs réservoirs selon l'invention, sur la ligne de lait.

Une première variante du procédé au niveau de l'étape d'ensemencement consiste à ensemencer le milieu lacté à traiter en une seule fois avec la suspension de ferments. Ceci est réalisé par vidange totale du ou des réservoir(s) scellé(s) en une seule fois. Il s'agit d'un ensemencement en lot (un seul réservoir) ou multi-lots (plusieurs réservoirs).

Une seconde variante du procédé au niveau de l'étape d'ensemencement consiste à ensemencer le milieu lacté à traiter de manière continue avec la suspension de micro-organismes.

Une troisième variante du procédé au niveau de l'étape d'ensemencement consiste à ensemencer le milieu lacté à traiter de manière discontinue avec la suspension de ferments.

Par discontinue on entend un cycle d'ensemencement réalisé de la manière suivante : on ensemence le milieu lacté à traiter pendant un laps de temps, puis on arrête l'ensemencement, puis on recommence l'ensemencement, ceci pendant plusieurs cycles.

Dans le cadre de cette troisième variante, l'ensemencement du milieu lacté à traiter avec la suspension de micro-organismes est réalisé à un débit compris entre 10 ml/min et 1000 ml/min, de préférence compris entre 100 ml/min et 500 ml/min, fait par intervalle régulier ou irrégulier de temps compris entre 1 minute et 600 minutes.

Les variantes décrites ci-dessus peuvent également être mises en oeuvre dans le cas de l'ensemencement d'un milieu carné ou d'un produit de salaison, par exemple de charcuterie, mais également et de manière plus générale dans l'industrie agro-alimentaire, pharmaceutique, cosmétique, alimentaire, l'agriculture, ainsi que dans les domaines de la nutrition animale, des aliments pour animaux de compagnies et des dentifrices ou compositions dentaires.

L'avantage de la mise en oeuvre du procédé selon l'invention grâce au réservoir d'ensemencement tel que décrit ci-dessus, est de réaliser un ensemencement direct, à température ambiante, stérile, standardisé, adaptable à chaque type de production et qui garantit la qualité bactériologique.

Un autre avantage de la mise en oeuvre du procédé selon l'invention grâce au réservoir d'ensemencement tel que décrit ci-dessus est de simplifier et fiabiliser l'étape d'ensemencement du ferment lactique.

Un autre avantage de la présente invention est de proposer un réservoir jetable ce qui réduit les risques de contamination par d'autres micro-organismes suite à une réutilisation des réservoirs. De plus cela évite la maintenance des cuves, limite les investissements et supprime l'étape de préparation d'un levain.

## Revendications

1. Procédé pour préparer une suspension de micro-organismes dans un liquide **caractérisé en ce que** l'on utilise un réservoir scellé (1) comprenant :
- 2 chambres communiquant par la partie Inférieure du réservoir,
- au moins un orifice (2) d'introduction d'un liquide dans une chambre,
- chaque chambre comprenant au moins un orifice (3) d'admission et au moins un orifice (4) d'évacuation d'un gaz
- le réservoir contenant en outre les microorganismes sous forme solide (8)
et **caractérisé en ce que** l'on introduit le liquide par l'orifice d'introduction dans au moins une des chambres, puis l'on introduit alternativement un gaz par lesdits orifices d'admission dans une chambre puis dans l'autre chambre de manière à créer un flux dudit liquide en va et vient dans chaque chambre.

2. Procédé selon la revendication 1 **caractérisé en ce qu'**on utilise un réservoir scellé comprenant 2 chambres disposées sensiblement en forme de U.

3. Procédé selon la revendication 2 **caractérisé en ce qu'**on utilise un réservoir scellé qui est une poche unique repliée sur elle-même de manière à former les 2 chambres (1a) et (1b).

4. Procédé selon l'une des revendications précédentes **caractérisé en ce qu'**on utilise un réservoir scellé (1) flexible ou souple, notamment en matière plastique.

5. Procédé selon l'une des revendications précédentes **caractérisé en ce qu'**on utilise un réservoir comprenant au moins un orifice (5) de remplissage permettant d'introduire les microorganismes sous forme solide (8) dans le réservoir.

6. Procédé selon l'une des revendications précédentes **caractérisé en ce qu'**on utilise un réservoir comprenant au moins un orifice (6) de vidange dudit réservoir.

7. Procédé selon l'une des revendications précédentes **caractérisé en ce qu'**on utilise des gaz tel que l'air, l'argon, l'azote, le dioxyde de carbone ou leurs mélanges.

8. Procédé selon l'une des revendications précédentes **caractérisé en ce qu'**on utilise un réservoir scellé fixé sur une potence (7), notamment une potence mobile.

9. Procédé d'ensemencement **caractérisé en ce qu'**il comprend
- la préparation selon l'une quelconque des revendications 1 à 8 d'une suspension de microorganismes et
- une étape d'ensemencement consistant à ensemencer un milieu lacté à traiter de manière continue avec la suspension de micro-organismes.

10. Procédé d'ensemencement **caractérisé en ce qu'**il comprend
- la préparation selon l'une quelconque des revendications 1 à 8 d'une suspension de microorganismes et
- une étape d'ensemencement consistant à ensemencer un milieu lacté à traiter en une seule fois ou de manière discontinue avec la suspension de micro-organismes,
et **en ce que** la suspension de microorganismes est une suspension de ferments.

## Claims

1. A method for preparing a suspension of microorganisms in a liquid, **characterized in that** a sealed reservoir (1) is used, comprising :
- 2 chambers that communicate by means of the lower section of the reservoir,
- at least one inlet opening (2) for introducing a liquid into a chamber,
- each chamber comprising at least one gas inlet opening (3) and at least one outlet opening (4) for evacuating a gas,
- the reservoir also containing the microorganisms in a solid form (8),
and **characterized in that** the liquid is introduced by means of the inlet opening into at least one of the chambers, then a gas is alternately introduced by means of said gas inlet openings into one chamber and then into the other chamber so as to generate an oscillating flow of said liquid in each chamber.

2. The method as claimed in claim 1, **characterized in that** a sealed reservoir comprising 2 chambers in a substantially U-shaped arrangement is used.

3. The method as claimed in claim 2, **characterized in that** a sealed reservoir that is a single pocket folded back on itself so as to form the 2 chambers (1a) and (1b) is used.

4. The method as claimed in one of the preceding claims, **characterized in that** a flexible or supple sealed reservoir (1), in particular made of plastic, is used.

5. The method as claimed in one of the preceding claims, **characterized in that** a reservoir comprising at least one filling opening (5) for introducing the microorganisms in the form of a solid (8) into the reservoir is used.

6. The method as claimed in one of the preceding claims, **characterized in that** a reservoir comprising at least one opening (6) for emptying said reservoir is used.

7. The method as claimed in one of the preceding claims, **characterized in that** gases such as air, argon, nitrogen or carbon dioxide, or mixtures thereof, are used.

8. The method as claimed in one of the preceding claims, **characterized in that** a sealed reservoir attached to a support bracket (7), in a particular a mobile support bracket, is used.

9. A method of seeding, **characterized in that** it comprises
- preparing, according to any of claims 1 to 8, a suspension of microorganisms, and
- a step of seeding consisting in seeding a milk medium to be treated, with the suspension of microorganisms, continuously.

10. A method of seeding **characterized in that** it comprises
- preparing, according to any of claims 1 to 8, a suspension of microorganisms, and
- a step of seeding consisting in seeding a milk medium to be treated, with the suspension of microorganisms, in a single step or discontinuously, and **in that** the suspension of microorganisms is a suspension of ferments.

## Patentansprüche

1. Verfahren zur Herstellung einer Suspension von Mikroorganismen in einer Flüssigkeit, **dadurch gekennzeichnet, dass** ein verschlossener Behälter (1) verwendet wird, umfassend:
• 2 Kammern, die über den unteren Abschnitt des Behälters in Verbindung stehen
• wenigstens eine Öffnung (2) für die Zufuhr einer Flüssigkeit in eine Kammer
• wobei jede Kammer wenigstens eine Einlassöffnung (3) und wenigstens eine Auslassöffnung (4) für ein Gas umfasst
• wobei der Behälter außerdem die Mikroorganismen in festem Zustand (8) enthält,
und **dadurch gekennzeichnet, dass** die Flüssigkeit über die Zufuhröffnung in wenigstens eine der Kammern zugeführt wird, dann über die Einlassöffnungen abwechselnd ein Gas in eine Kammer, dann in die andere Kammer zugeführt wird, sodass in jeder der Kammern eine hin- und her bewegende Strömung der Flüssigkeit erzeugt wird.

2. Verfahren nach Anspruch 1, **dadurch gekennzeichnet, dass** ein verschlossener Behälter verwendet wird, der 2 im Wesentlichen U-förmig angeordnete Kammern umfasst.

3. Verfahren nach Anspruch 2, **dadurch gekennzeichnet, dass** ein verschlossener Behälter verwendet wird, der eine einzelne Tasche ist, die so auf sich zusammengefaltet ist, dass die 2 Kammern (1a) und (1b) geformt werden.

4. Verfahren nach einem der vorhergehenden Ansprüche, **dadurch gekennzeichnet, dass** ein flexibler oder biegsamer verschlossener Behälter (1) verwendet wird, insbesondere aus Kunststoffmaterial.

5. Verfahren nach einem der vorhergehenden Ansprüche, **dadurch gekennzeichnet, dass** ein Behälter verwendet wird, der wenigstens eine Einfüllöffnung (5) umfasst, die das Einbringen der Mikroorganismen in fester Form (8) in den Behälter ermöglicht.

6. Verfahren nach einem der vorhergehenden Ansprüche, **dadurch gekennzeichnet, dass** ein Behälter verwendet wird, der wenigstens eine Abflussöffnung (6) aus dem Behälter umfasst.

7. Verfahren nach einem der vorhergehenden Ansprüche, **dadurch gekennzeichnet, dass** Gase wie etwa Luft, Argon, Stickstoff, Kohlendioxid oder Gemische davon verwendet werden.

8. Verfahren nach einem der vorhergehenden Ansprüche, **dadurch gekennzeichnet, dass** ein auf einem Träger (7), insbesondere einem beweglichen Träger, befestigter verschlossener Behälter verwendet wird.

9. Aussaatverfahren, **dadurch gekennzeichnet, dass** es umfasst:
• die Herstellung einer Suspension von Mikroorganismen nach einem der Ansprüche 1 bis 8, und
• einen Aussaatschritt, der aus der kontinuierlichen Aussaat der Suspension von Mikroorganismen auf einem Laktatmedium besteht.

10. Aussaatverfahren, **dadurch gekennzeichnet, dass** es umfasst:
• die Herstellung einer Suspension von Mikroorganismen nach einem der Ansprüche 1 bis 8, und
• einen Aussaatschritt, der aus der einmaligen oder diskontinuierlichen Aussaat der Suspension von Mikroorganismen auf einem Laktatmedium besteht,
und wobei die Suspension von Mikroorganismen eine Fermentationsuspension ist.
